# EUROPEAN PATENT APPLICATION

(11) **EP 4 154 931 A1**
(43) Date of publication of application: **29.03.2023**
(21) Application number: 20965431.8
(22) Date of filing: 16.12.2020
(51) Int. Cl.: A61M 16/01, A61M 16/22

(54) **ANESTHESIA MACHINE, VETERINARY ANESTHESIA MACHINE, AND EXHAUST GAS ABSORPTION TANK SUPPORT APPARATUS THEREOF**

(71) Applicant: Shenzhen Mindray Animal Medical Technology Co., Ltd., Shenzhen, Guangdong 518110 (CN)
(72) Inventor: SONG, Juntao, Shenzhen, Guangdong 518057 (CN); WANG, Xuan, Shenzhen, Guangdong 518057 (CN)
(74) Representative: KIPA AB
(86) International application number: PCT/CN2020/136727
(87) International publication number: WO 2022/126413

(57) **Abstract**

The disclosure provides an anesthesia machine, a veterinary anesthesia machine, and a waste gas absorption tank supporting device, the waste gas absorption tank supporting device including: a tray provided with a tray recess; and a protrusion structure provided on the tray, the protrusion structure being capable of forming a circumferential protective structure located on an outer side of the tray recess and configured to protect a waste gas absorption tank, and an inner side surface and an outer side surface of the protrusion structure being both protective surfaces capable of protecting the waste gas absorption tank. The waste gas absorption tank supporting device provided by the disclosure can effectively support waste gas absorption tanks of various sizes, so that the waste gas absorption tank is prevented from sliding off from the waste gas absorption tank supporting device during the movement of the anesthesia machine, and the universality of the waste gas absorption tank supporting device is effectively improved.

## Description

### TECHNICAL FIELD

The disclosure relates to the technical field of anesthesia machine apparatuses, and in particular to an anesthesia machine, a veterinary anesthesia machine, and a waste gas absorption tank supporting device therefor.

### BACKGROUND

At present, a waste gas absorption tank provided on an anesthesia machine needs to be supported by a waste gas absorption tank supporting device. However, only waste gas absorption tanks of common sizes can be effectively supported at present, while it is impossible for waste gas absorption tanks of other sizes after being provided on the waste gas absorption tank supporting device, due to their sizes, to avoid sliding off of the waste gas absorption tanks from the waste gas absorption tank supporting device during the movement of the anesthesia machine, and the waste gas absorption tank supporting device is not universal.

Therefore, the technical problems to be currently solved by a person skilled in the art are how to prevent a waste gas absorption tank from sliding off and to improve the universality.

### SUMMARY

An object of the disclosure is to provide a waste gas absorption tank supporting device, so as to prevent a waste gas absorption tank from sliding off and to improve universality. The disclosure further provides an anesthesia machine and a veterinary anesthesia machine having the above-mentioned waste gas absorption tank supporting device.

In order to solve the above technical problems, the disclosure provides a waste gas absorption tank supporting device, including:
a tray provided with a tray recess; and
a protrusion structure provided on the tray, the protrusion structure being capable of forming a circumferential protective structure located on an outer side of the tray recess and configured to protect a waste gas absorption tank, and an inner side surface and an outer side surface of the protrusion structure being both protective surfaces capable of protecting the waste gas absorption tank.

The disclosure further provides an anesthesia machine including the waste gas absorption tank supporting device described above.

According to the waste gas absorption tank supporting device provided by the disclosure, when it is required to provide a waste gas absorption tank having a diameter less than or equal to that of the tray recess on the waste gas absorption tank supporting device, the waste gas absorption tank can be embedded into the tray recess in such a way that there is a certain gap or fit between an inner side wall of the tray recess and an outer side surface of the waste gas absorption tank, thereby protecting the waste gas absorption tank by the inner side wall of the tray recess, and preventing the waste gas absorption tank from sliding off during the movement of the anesthesia machine. When it is required to provide a waste gas absorption tank having a diameter greater than that of the tray recess on the waste gas absorption tank supporting device, and when the circumferential protective structure can surround the outside of the waste gas absorption tank, the inner side surface of the protrusion structure forms a protective surface for protecting the waste gas absorption tank, wherein there is a certain gap or fit between the inner side surface of the protrusion structure and the outer side surface of the waste gas absorption tank, thereby protecting the waste gas absorption tank by an inner side wall of the protrusion structure, and is preventing the waste gas absorption tank from sliding off during the movement of the anesthesia machine. When it is required to provide a waste gas absorption tank having a diameter greater than that of the tray recess on the waste gas absorption tank supporting device, and when the circumferential protective structure is located in a bottom trough body of the waste gas absorption tank, the outer side surface of the protrusion structure forms a protective surface for protecting the waste gas absorption tank, wherein the outer side surface of the protrusion structure is fitted with an inner side wall of the bottom trough body of the waste gas absorption tank, thereby protecting the waste gas absorption tank by the outer side surface of the protrusion structure, and preventing the waste gas absorption tank from sliding off during the movement of the anesthesia machine. When it is required to provide a waste gas absorption tank having a diameter greater than that of the tray recess on the waste gas absorption tank supporting device, and when the circumferential protective structure is located in a bottom trough body of the waste gas absorption tank, the outer side surface of the protrusion structure forms a protective surface for protecting the waste gas absorption tank, wherein there is a certain gap between the outer side surface of the protrusion structure and an inner side wall of the bottom trough body of the waste gas absorption tank, thereby protecting the waste gas absorption tank by the outer side surface of the protrusion structure, and preventing the waste gas absorption tank from sliding off during the movement of the anesthesia machine. The waste gas absorption tank supporting device provided by the disclosure can effectively support waste gas absorption tanks of various sizes, so that the waste gas absorption tank is prevented from sliding off from the waste gas absorption tank supporting device during the movement of the anesthesia machine, and the universality of the waste gas absorption tank supporting device is effectively improved.

The disclosure further provides an anesthesia machine having the above-mentioned waste gas absorption tank supporting device. Since the above-mentioned waste gas absorption tank supporting device has the above technical effect, the anesthesia machine having the above-mentioned waste gas absorption tank supporting device should also have the same technical effect, which will not be described in detail herein.

### BRIEF DESCRIPTION OF THE DRAWINGS

FIG. 1 is a first schematic structural diagram of a first waste gas absorption tank supporting device provided by the disclosure;
FIG. 2 is a first schematic cross-sectional view of the first waste gas absorption tank supporting device provided by the disclosure;
FIG. 3 is a second schematic cross-sectional view of the first waste gas absorption tank supporting device provided by the disclosure;
FIG. 4 is a third schematic cross-sectional view of the first waste gas absorption tank supporting device provided by the disclosure;
FIG. 5 is a fourth schematic cross-sectional view of the first waste gas absorption tank supporting device provided by the disclosure;
FIG. 6 is a first schematic cross-sectional view of a second waste gas absorption tank supporting device provided by the disclosure;
FIG. 7 is a second schematic cross-sectional view of the second waste gas absorption tank supporting device provided by the disclosure; and
FIG. 8 is a schematic structural diagram of a third waste gas absorption tank supporting device provided by the disclosure.

### DESCRIPTION OF THE PREFERRED EMBODIMENTS

The core of the disclosure is to provide a waste gas absorption tank supporting device, so as to prevent a waste gas absorption tank from sliding off and to improve the universality. The disclosure further provides an anesthesia machine and a veterinary anesthesia machine having the above-mentioned waste gas absorption tank supporting device.

In order to better understand the solutions of the disclosure by those skilled in the art, the following detailed description of the disclosure is made with reference to the accompanying drawings and particular embodiments.

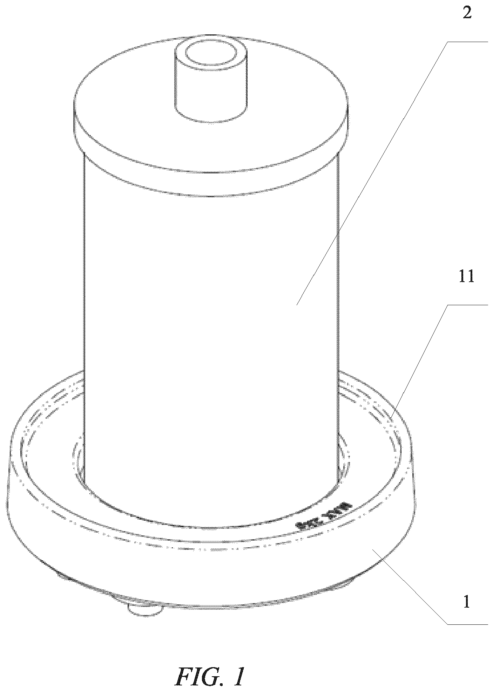

Referring to FIGS. 1 to 5, a waste gas absorption tank supporting device provided by the disclosure includes a tray 1 and a protrusion structure 11 provided on the tray 1. The tray 1 is provided with a tray recess 12; and the protrusion structure 11 is capable of forming a circumferential protective structure located on an outer side of the tray recess 12 and configured to protect a waste gas absorption tank 2, and an inner side surface and an outer side surface of the protrusion structure 11 are both protective surfaces capable of protecting the waste gas absorption tank.

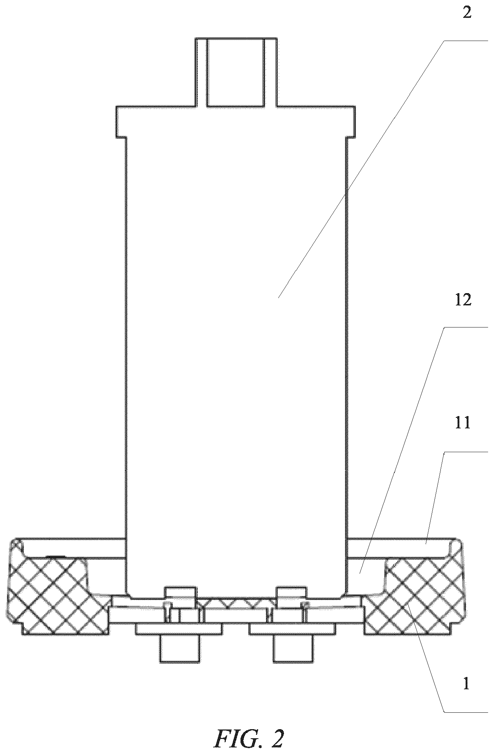

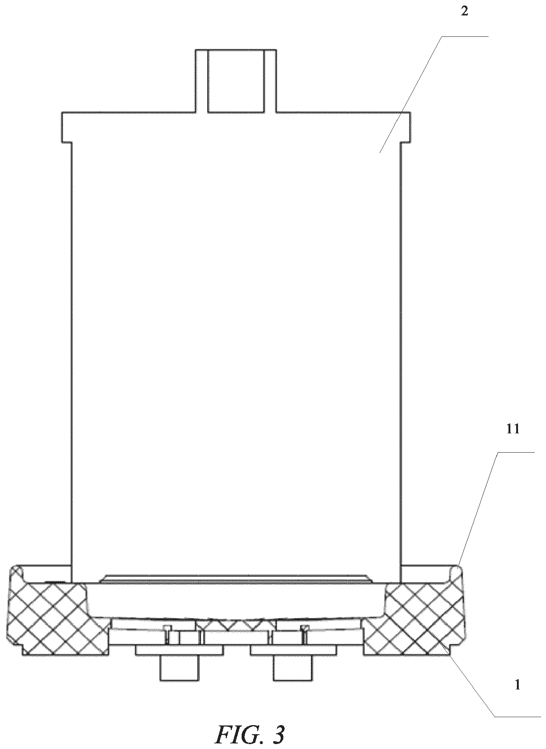

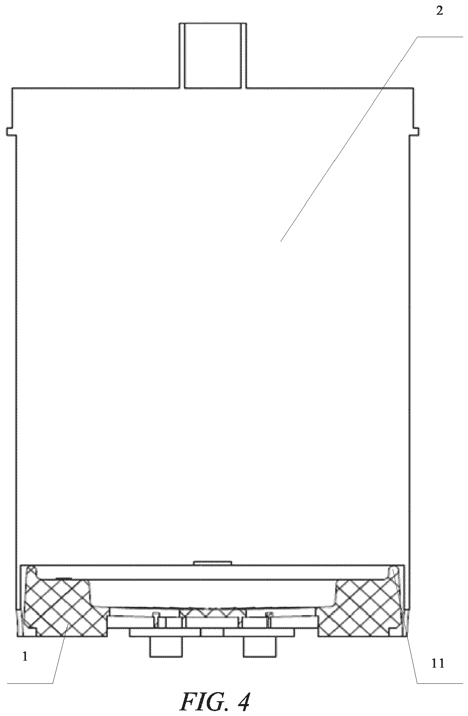

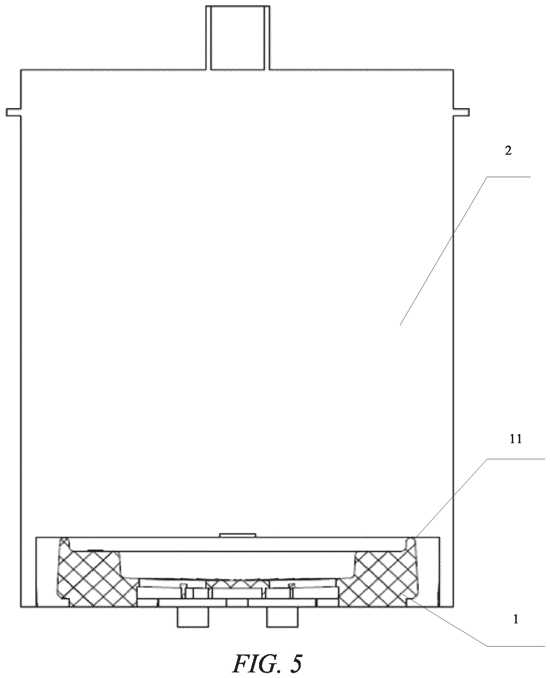

As shown in FIG. 1 and FIG. 2, when it is required to provide the waste gas absorption tank 2 having a diameter less than or equal to that of the tray recess 12 on the waste gas absorption tank supporting device, the waste gas absorption tank 2 can be embedded into the tray recess 12 in such a way that there is a certain gap or fit between an inner side wall of the tray recess 12 and an outer side surface of the waste gas absorption tank 2, thereby protecting the waste gas absorption tank 2 by the inner side wall of the tray recess 12, and preventing the waste gas absorption tank 2 from sliding off during the movement of the anesthesia machine. As shown in FIG. 3, when it is required to provide the waste gas absorption tank 2 having a diameter greater than that of the tray recess 12 on the waste gas absorption tank supporting device, and when the circumferential protective structure can surround the outside of the waste gas absorption tank 2, the inner side surface of the protrusion structure 11 forms a protective surface for protecting the waste gas absorption tank, wherein there is a certain gap or fit between the inner side surface of the protrusion structure 11 and the outer side surface of the waste gas absorption tank 2, thereby protecting the waste gas absorption tank 2 by an inner side wall of the protrusion structure 11, and preventing the waste gas absorption tank 2 from sliding off during the movement of the anesthesia machine. As shown in FIG. 4, when it is required to provide the waste gas absorption tank 2 having a diameter greater than that of the tray recess 12 on the waste gas absorption tank supporting device, and when the circumferential protective structure is located in a bottom trough body of the waste gas absorption tank 2, the outer side surface of the protrusion structure 11 forms a protective surface for protecting the waste gas absorption tank, wherein the outer side surface of the protrusion structure 11 is fitted with an inner side wall of the bottom trough body of the waste gas absorption tank 2, thereby protecting the waste gas absorption tank 2 by the outer side surface of the protrusion structure 11, and preventing the waste gas absorption tank 2 from sliding off during the movement of the anesthesia machine. As shown in FIG. 5, when it is required to provide the waste gas absorption tank 2 having a diameter greater than that of the tray recess 12 on the waste gas absorption tank supporting device, and when the circumferential protective structure is located in a bottom trough body of the waste gas absorption tank 2, the outer side surface of the protrusion structure 11 forms a protective surface for protecting the waste gas absorption tank, wherein there is a certain gap between the outer side surface of the protrusion structure 11 and an inner side wall of the bottom trough body of the waste gas absorption tank 2, thereby protecting the waste gas absorption tank 2 by the outer side surface of the protrusion structure 11, and preventing the waste gas absorption tank 2 from sliding off during the movement of the anesthesia machine.

Therefore, the waste gas absorption tank supporting device provided in the embodiments of the disclosure can effectively support waste gas absorption tanks of various sizes, so that the waste gas absorption tank 2 is prevented from sliding off from the waste gas absorption tank supporting device during the movement of the anesthesia machine, and the universality of the waste gas absorption tank supporting device is effectively improved.

In this embodiment, the protrusion structure 11 is an annular flange. That is, the annular flange itself forms the circumferential protective structure for protecting the waste gas absorption tank 2. Preferably, the annular flange and the tray recess 12 are arranged in a concentric circle configuration.

In order to improve effective support for a large-sized waste gas absorption tank 2, an outer side surface of the annular flange is aligned with an outer circumferential surface of the tray 1. As shown in FIGS. 4 and 5, when the circumferential protective structure is located in a bottom trough body of the waste gas absorption tank 2, since the outer side surface of the annular flange is aligned with the outer circumferential surface of the tray 1, when the depth of the bottom trough body of the waste gas absorption tank 2 allows adjustment, there is a certain gap or fit both between the outer side surface of the annular flange and the inner side wall of the bottom trough body of the waste gas absorption tank 2 and between the outer circumferential surface of the tray 1 and the inner side wall of the bottom trough body of the waste gas absorption tank 2, thereby effectively improving the supporting stability of the waste gas absorption tank supporting device to the waste gas absorption tank 2.

Of course, it is also possible to have a predetermined dimension between the outer side surface of the annular flange and the outer circumferential surface of the tray 1, which is not described in detail herein and falls within the scope of protection.

In another embodiment, a plurality of protrusion structures 11 are provided and arranged in a circumferential direction of the tray 1. That is, the plurality of protrusion structures 11 are arranged in an annular ring. Each protrusion structure 11 may be a square protrusion, a circular protrusion, a triangular protrusion, or the like.

In order to avoid damages to an outer wall of the waste gas absorption tank 2, the protrusion structure 11 is preferably made of a flexible material. The flexible material may be silicone or rubber, etc.

The protrusion structure 11 may be fixed relative to the tray 1.

The protrusion structure 11 is fixedly connected to the tray 1 by assembly. The protrusion structure 11 is manufactured separately from the tray 1, and the protrusion structure 11 is then assembled and fixed to the tray 1 by means of a connector such as a fastener or a bolt.

The protrusion structure 11 may be connected to the tray 1 by means of a fixing bolt. That is, the protrusion structure 11 is provided with a mounting hole, and the tray 1 is provided with a threaded hole; and the waste gas absorption tank supporting device provided in this embodiment further includes a fixing screw that passes through the mounting hole to be in thread engagement with the threaded hole. With the above arrangement, the assembly and fixing of the protrusion structure 11 and the tray 1 can be achieved by installing the fixing screw only on the side facing a top surface of the protrusion structure 11, so that the assembly and fixing operations are facilitated. It will be appreciated that the top surface of the protrusion structure 11 is a side thereof facing away from the tray 1.

Of course, it is also possible to connect the protrusion structure 11 to the tray 1 by means of a fastener structure. They may also be connected by means of a positioning pin or other components. The connection of the protrusion structure 11 to the tray 1 may also be achieved by connection manners such as welding, riveting, hot melting or adhesion. Alternatively, instead of the threaded hole in the tray 1, an unthreaded hole is provided, and a nut is further included, the fixing screw being in thread engagement with the nut after passing through the mounting hole and the unthreaded hole.

They are not repeated in detail herein and fall within the scope of protection.

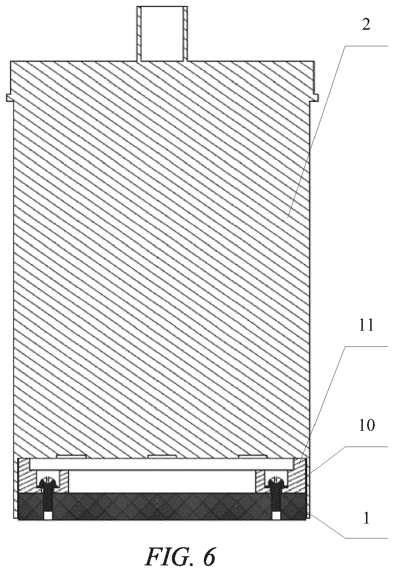

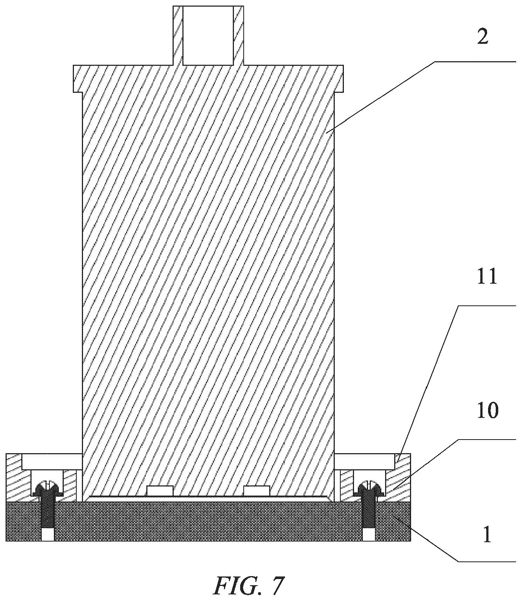

As shown in FIGS. 6 and 7, in this embodiment, a connecting block 10 is provided on the tray 1; and an inner wall of the connecting block 10 close to the center of the tray 1 is the inner side wall of the tray recess 12, and the protrusion structure 11 is a part of the connecting block 10 away from the center of the tray 1. That is, by providing the connecting block 10 on the tray 1, it is possible to achieve an operation of providing both the tray recess 12 and the protrusion structure 11 on the tray 1. Thus, the surface of the tray 1 for providing the connecting block 10 may be a plane or the like. It is not required to machine the tray recess 12 on the surface of the tray 1 provided with the connecting block 10, so that machining of the tray 1 is facilitated.

The connecting block 10 may be an annular member, and in order to achieve the above-mentioned effect, the connecting block 10 is provided with a stepped hole; the stepped hole includes a primary inner hole and a secondary inner hole, and the primary inner hole has a smaller dimension than the secondary inner hole; a hole wall of the primary inner hole is the inner side wall of the tray recess 12; and a hole wall of the secondary inner hole, a top wall of the connecting block 10 and an outer wall of the connecting block 10 form the protrusion structure 11. The hole wall of the secondary inner hole, the top wall of the connecting block 10 and the outer wall of the connecting block 10 are sequentially connected to form a structure protruding away from the tray 1, and the structure is the protrusion structure 11.

As shown in FIG. 6, when it is required to provide the waste gas absorption tank 2 having a diameter greater than that of the tray recess 12 on the waste gas absorption tank supporting device, and when the circumferential protective structure formed by the protrusion structure 11 is located in the bottom trough body of the waste gas absorption tank 2, the outer wall of the connecting block 10 (the outer side surface of the protrusion structure 11) or a circumferential outer wall of the tray 1 forms a protective surface for protecting the waste gas absorption tank, wherein the outer wall of the connecting block 10 or the circumferential outer wall of the tray 1 is fitted with or has a certain gap with the inner side wall of the bottom trough body of the waste gas absorption tank 2, thereby protecting the waste gas absorption tank 2 by the outer wall of the connecting block 10 or the circumferential outer wall of the tray 1, and preventing the waste gas absorption tank 2 from slipping off during the movement of the anesthesia machine.

As shown in FIG. 7, when it is required to provide the waste gas absorption tank 2 having a diameter less than or equal to that of the tray recess 12 on the waste gas absorption tank supporting device, the waste gas absorption tank 2 can be embedded into the tray recess 12 in such a way that there is a certain gap or fit between the inner side wall of the tray recess 12 and the outer side surface of the waste gas absorption tank 2, thereby protecting the waste gas absorption tank 2 by the inner side wall of the tray recess 12 (the hole wall of the primary inner hole), and preventing the waste gas absorption tank 2 from sliding off during the movement of the anesthesia machine.

In addition, when it is required to provide the waste gas absorption tank 2 having a diameter greater than that of the tray recess 12 on the waste gas absorption tank supporting device, and when the circumferential protective structure can surround the outside of the waste gas absorption tank 2, the hole wall of the secondary inner hole (the inner side surface of the protrusion structure 11) forms a protective surface for protecting the waste gas absorption tank, wherein there is a certain gap or fit between the hole wall of the secondary inner hole and the outer side surface of the waste gas absorption tank 2, thereby protecting the waste gas absorption tank 2 by the hole wall of the secondary inner hole, and preventing the waste gas absorption tank 2 from sliding off during the movement of the anesthesia machine.

In this embodiment, a counter bore is formed in a connection plane between the primary inner hole and the secondary inner hole, and a threaded hole is formed in the tray 1. The waste gas absorption tank supporting device further includes a fixing screw that passes through the counter bore to be in thread engagement with the threaded hole. With the above arrangement, the fixed connection of the connecting block 10 to the tray 1 is achieved. With the above arrangement, the assembly and fixing of the connecting block 10 and the tray 1 can be achieved by installing the fixing screw only on a side facing a top surface of the connecting block 10, so that the assembly and fixing operations are facilitated. It will be appreciated that the top surface of the connecting block 10 is a side thereof facing away from the tray 1.

A gasket may be provided in the counter bore. A head portion of the fixing screw is inserted into the counter bore, which not only plays a connection role, but also prevents the head portion of the fixing screw from being exposed to interfere with the waste gas absorption tank 2.

It is also possible to provide a counter bore in the tray 1, a threaded hole is formed in the connecting block 10, and the fixing screw passes through the counter bore and is in thread engagement with the threaded hole to achieve the fixed connection between the connecting block 10 and the tray 1.

Of course, it is also possible to replace the counter bore with an unthreaded hole structure only for a stud portion of the fixing screw to pass through.

It is also possible to connect the connecting block 10 to the tray 1 by means of a fastener structure. They may also be connected by means of a positioning pin or other components. Alternatively, the connection of the connecting block 10 to the tray 1 may also be achieved by connection manners such as welding, riveting, hot melting or adhesion. Alternatively, instead of the threaded hole in the tray 1, an unthreaded hole is provided, and a nut is further included, the fixing screw being in thread engagement with the nut after passing through the mounting hole and the unthreaded hole.

A plurality of connecting blocks 10 may also be provided, the connecting blocks 10 may be independent structures such as block structures or strip structures, and the plurality of connecting blocks 10 may be arranged in the circumferential direction of the tray 1 to form an annular arrangement structure in which the plurality of connecting blocks 10 are arranged. Each connecting block 10 includes a primary face and a secondary face that are arranged in a direction away from the center of the tray 1, wherein the primary face and the secondary face are located on a side of the connecting block 10 facing away from the tray 1; each connecting block 10 further includes a primary facade connected to an end of the primary face away from the secondary face, and a secondary facade connecting the primary face to the secondary face, wherein the primary facade and the secondary facade face the center of the tray 1; the primary facade is the inner side wall of the tray recess 12; and the secondary facade, the secondary face and the outer wall of the connecting block 10 form the protrusion structure 11.

Further, a counter bore is formed in the primary face, and a threaded hole is formed in the tray 1; and a fixing screw is also included, the fixing screw passing through the counter bore to be in thread engagement with the threaded hole. With the above arrangement, the fixed connection of the connecting block 10 to the tray 1 is achieved. With the above arrangement, the assembly and fixing of the connecting block 10 and the tray 1 can be achieved by installing the fixing screw only on a side facing a top surface of the connecting block 10, so that the assembly and fixing operations are facilitated. It will be appreciated that the top surface of the connecting block 10 is a side thereof facing away from the tray 1.

Of course, it is also possible to connect the connecting block 10 to the tray 1 by means of a fastener structure. They may also be connected by means of a positioning pin or other components. The connection of the connecting block 10 to the tray 1 may also be achieved by connection manners such as welding, riveting, hot melting or adhesion. Alternatively, instead of the threaded hole in the tray 1, an unthreaded hole is provided, and a nut is further included, the fixing screw being in thread engagement with the nut after passing through the mounting hole and the unthreaded hole.

A gasket may be provided in the counter bore. A head portion of the fixing screw is inserted into the counter bore, which not only plays a connection role, but also prevents the head portion of the fixing screw from being exposed to interfere with the waste gas absorption tank 2.

It is also possible to provide a counter bore in the tray 1, a threaded hole is formed in the connecting block 10, and the fixing screw passes through the counter bore and is in thread engagement with the threaded hole to achieve the fixed connection between the connecting block 10 and the tray 1. In another embodiment, the protrusion structure 11 and the tray 1 are integrated with each other. As shown in FIGS. 1 to 5, the protrusion structure 11 and the tray 1 may be integrated with each other by means of injection molding or turning.

It is also possible that the protrusion structure 11 is movably arranged on the tray 1. When it is required to provide the waste gas absorption tank 2 having a diameter greater than that of the tray recess 12 on the waste gas absorption tank supporting device, the protrusion structure 11 is as close as possible to the waste gas absorption tank 2 by adjusting the position of the protrusion structure 11 on the tray 1, so as to improve a protective effect.

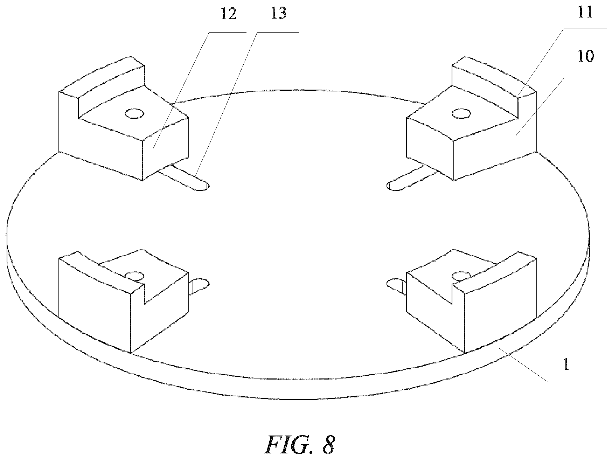

As shown in FIG. 8, a plurality of sliding slots 13 are provided in the tray 1, and extension directions of the sliding slots 13 are oriented in a radial direction of the tray 1; and a plurality of protrusion structures 11 are provided and arranged in the circumferential direction of the tray 1, and the protrusion structures 11 are in one-to-one sliding fit with the sliding slots. The positions of the protrusion structures 11 on the tray 1 are adjusted by the sliding of the protrusion structures 11 along the sliding slots 13, such that the protrusion structures 11 are as close as possible to the waste gas absorption tank 2. It is also possible to provide arc-shaped grooves or V-shaped grooves or the like in the tray 1, which will not be described in detail herein.

A connecting block 10 is provided on the tray 1; the connecting block 10 includes a primary face and a secondary face that are arranged in a direction away from the center of the tray 1, wherein the primary face and the secondary face are located on a side of the connecting block 10 facing away from the tray 1; the connecting block 10 further includes a primary facade connected to an end of the primary face away from the secondary face, and a secondary facade connecting the primary face to the secondary face, wherein the primary facade and the secondary facade face the center of the tray 1; the primary facade is the inner side wall of the tray recess 12; and the secondary facade, the secondary face and the outer wall of the connecting block 10 form the protrusion structure 11. Thus, the surface of the tray 1 for providing the connecting block 10 may be a plane or the like. It is not required to machine the tray recess 12 on the surface of the tray 1 provided with the connecting block 10, so that machining of the tray 1 is facilitated.

In order to facilitate machining, a counter bore is formed in the primary face; and a sliding bolt mounted in the counter bore is further included, and a stud of the sliding bolt is slidably disposed in the corresponding sliding slot 13.

A gasket may be provided in the counter bore. A head portion of the sliding bolt is embedded into the counter bore, which not only plays a sliding connection role, but also prevents the head portion of the sliding bolt from being exposed to interfere with the waste gas absorption tank 2.

It is also possible to provide a sliding rail on the tray 1, and the protrusion structure 11 is slidably disposed on the sliding rail in the form of a slider. As shown in FIG. 8, taking the example that four protrusion structures 11 are uniformly arranged in the circumferential direction of the tray 1, a cross track group including two sliding rails may be provided, and two oppositely provided protrusion structures 11 are provided on the same sliding rail.

An embodiment of the disclosure further provides an anesthesia machine, including a fresh gas circuit, a driving gas circuit, an anesthetic gas recovery branch, a patient's inspiratory branch and a patient's expiratory branch,
wherein the driving gas circuit includes a gas driving device configured to force a driving gas into the patient's inspiratory branch;
the fresh gas circuit includes an anesthetic evaporation tank, a fresh gas carries an anesthetic gas in the anesthetic evaporation tank to the patient's inspiratory branch via the fresh gas circuit, to be mixed with the driving gas to form a mixed gas which is then delivered to a patient side; and
the anesthetic gas recovery branch is connected to the patient's expiratory branch, a waste anesthetic gas exhaled from the patient side enters a waste anesthetic gas recovery branch via the patient's expiratory branch, and the waste anesthetic gas recovery branch includes a waste anesthetic gas absorption tank and a waste gas absorption tank supporting device, the waste gas absorption tank supporting device being any one of the above-mentioned waste gas absorption tank supporting devices. That is, the waste gas absorption tank supporting device includes:
   a tray provided with a tray recess; and
   a protrusion structure provided on the tray, the protrusion structure being capable of forming a circumferential protective structure located on an outer side of the tray recess and configured to protect the waste gas absorption tank, and an inner side surface and an outer side surface of the protrusion structure being both protective surfaces capable of protecting the waste gas absorption tank.

Since the above-mentioned waste gas absorption tank supporting device has the above technical effect, the anesthesia machine having the above-mentioned waste gas absorption tank supporting device should also have the same technical effect, which will not be described in detail herein.

Further, the anesthesia machine in this embodiment may be a veterinary anesthesia machine. Namely, an embodiment of the disclosure further provides a veterinary anesthesia machine, the anesthesia machine including a fresh gas circuit, a driving gas circuit, an anesthetic gas recovery branch, a veterinary inspiratory branch and a veterinary expiratory branch,
wherein the driving gas circuit includes a gas driving device configured to force a driving gas into the veterinary inspiratory branch;
the fresh gas circuit includes an anesthetic evaporation tank, a fresh gas carries an anesthetic gas in the anesthetic evaporation tank to the veterinary inspiratory branch via the fresh gas circuit, to be mixed with the driving gas to form a mixed gas which is then delivered to a patient side; and
the anesthetic gas recovery branch is connected to the veterinary expiratory branch, a waste anesthetic gas exhaled by the patient side enters a waste anesthetic gas recovery branch via the veterinary expiratory branch, and the waste anesthetic gas recovery branch includes a waste anesthetic gas absorption tank and a waste gas absorption tank supporting device;
wherein the waste gas absorption tank supporting device includes:
   a tray provided with a tray recess; and
   a protrusion structure provided on the tray, the protrusion structure being capable of forming a circumferential protective structure located on an outer side of the tray recess and configured to protect the waste gas absorption tank, and an inner side surface and an outer side surface of the protrusion structure being both protective surfaces capable of protecting the waste gas absorption tank.

The waste gas absorption tank supporting device provided by the disclosure is described in detail above. Specific examples are used herein to explain the principles and implementation of the disclosure, and the above description of the embodiments is merely used to facilitate understanding of the method of the disclosure and the core concept thereof. It should be noted that for a person of ordinary skill in the art, numerous improvements and modifications may also be made to the disclosure without departing from the principles of the disclosure. Such improvements and modifications also fall within the scope of protection of the appended claims of the disclosure.

## Claims

1. An anesthesia machine, comprising a fresh gas circuit, a driving gas circuit, an anesthetic gas recovery branch, a patient's inspiratory branch and a patient's expiratory branch,
wherein the driving gas circuit comprises a gas driving device configured to force a driving gas into the patient's inspiratory branch;
the fresh gas circuit comprises an anesthetic evaporation tank, a fresh gas carries an anesthetic gas in the anesthetic evaporation tank to the patient's inspiratory branch via the fresh gas circuit, to be mixed with the driving gas to form a mixed gas which is then delivered to a patient side; and
the anesthetic gas recovery branch is connected to the patient's expiratory branch, a waste anesthetic gas exhaled by the patient side enters a waste anesthetic gas recovery branch via the patient's expiratory branch, and the waste anesthetic gas recovery branch comprises a waste anesthetic gas absorption tank and a waste gas absorption tank supporting device;
wherein the waste gas absorption tank supporting device comprises:
a tray provided with a tray recess; and
a protrusion structure provided on the tray, the protrusion structure being capable of forming a circumferential protective structure located on an outer side of the tray recess and configured to protect the waste gas absorption tank, and an inner side surface and an outer side surface of the protrusion structure being both protective surfaces capable of protecting the waste gas absorption tank.

2. The anesthesia machine according to claim 1, wherein the protrusion structure is an annular flange.

3. The anesthesia machine according to claim 2, wherein an outer side surface of the annular flange is aligned with an outer circumferential surface of the tray.

4. The anesthesia machine according to claim 2, wherein there is a predetermined dimension between an outer side surface of the annular flange and an outer circumferential surface of the tray.

5. The anesthesia machine according to claim 1, wherein a plurality of protrusion structures are provided and arranged in a circumferential direction of the tray.

6. The anesthesia machine according to claim 1, wherein the protrusion structure is made of a flexible material.

7. The anesthesia machine according to any one of claims 1-6, wherein the protrusion structure is fixed relative to the tray.

8. The anesthesia machine according to claim 7, wherein the protrusion structure is fixedly connected to the tray by assembly.

9. The anesthesia machine according to claim 8, wherein the protrusion structure is provided with a mounting hole, and the tray is provided with a threaded hole; and
a fixing screw is further comprised, the fixing screw passing through the mounting hole to be in thread engagement with the threaded hole.

10. The anesthesia machine according to claim 8, wherein a connecting block is provided on the tray; and
an inner wall of the connecting block close to the center of the tray is an inner side wall of the tray recess, and the protrusion structure is a part of the connecting block away from the center of the tray.

11. The anesthesia machine according to claim 10, wherein the connecting block is provided with a stepped hole;
the stepped hole comprises a primary inner hole and a secondary inner hole, and the primary inner hole has a smaller dimension than the secondary inner hole;
a hole wall of the primary inner hole is the inner side wall of the tray recess; and
a hole wall of the secondary inner hole, a top wall of the connecting block and an outer wall of the connecting block form the protrusion structure.

12. The anesthesia machine according to claim 11, wherein a counter bore is formed in a connection plane between the primary inner hole and the secondary inner hole, and a threaded hole is formed in the tray; and
a fixing screw is further comprised, the fixing screw passing through the counter hole to be in thread engagement with the threaded hole.

13. The anesthesia machine according to claim 10, wherein a plurality of connecting blocks are provided and arranged in a circumferential direction of the tray;
each connecting block comprises a primary face and a secondary face that are arranged in a direction away from the center of the tray, the primary face and the secondary face being located on a side of the connecting block facing away from the tray;
each connecting block further comprises a primary facade connected to an end of the primary face away from the secondary face, and a secondary facade connecting the primary face to the secondary face, wherein the primary facade and the secondary facade face the center of the tray;
the primary facade is the inner side wall of the tray recess; and
the secondary facade, the secondary face and an outer wall of the connecting block form the protrusion structure.

14. The anesthesia machine according to claim 13, wherein a counter bore is formed in the primary face, and a threaded hole is formed in the tray; and
a fixing screw is further comprised, the fixing screw passing through the counter hole to be in thread engagement with the threaded hole.

15. The anesthesia machine according to claim 7, wherein the protrusion structure and the tray are integrated with each other.

16. The anesthesia machine according to any one of claims 1-6, wherein the protrusion structure is movably arranged on the tray.

17. The anesthesia machine according to claim 16, wherein a plurality of sliding slots are provided in the tray, and extension directions of the sliding slots are oriented in a radial direction of the tray; and
a plurality of protrusion structures are provided and arranged in a circumferential direction of the tray, and the protrusion structures are in one-to-one sliding fit with the sliding slots.

18. The anesthesia machine according to claim 17, wherein a connecting block is provided on the tray;
the connecting block comprises a primary face and a secondary face that are arranged in a direction away from the center of the tray, the primary face and the secondary face being located on a side of the connecting block facing away from the tray;
the connecting block further comprises a primary facade connected to an end of the primary face away from the secondary face, and a secondary facade connecting the primary face to the secondary face, wherein the primary facade and the secondary facade face the center of the tray;
the primary facade is the inner side wall of the tray recess; and
the secondary facade, the secondary face and an outer wall of the connecting block form the protrusion structure.

19. The anesthesia machine according to claim 18, wherein a counter bore is formed in the primary face; and
a sliding bolt mounted in the counter bore is further comprised, and a stud of the sliding bolt is slidably disposed in the corresponding sliding slot.

20. A veterinary anesthesia machine, comprising a fresh gas circuit, a driving gas circuit, an anesthetic gas recovery branch, a veterinary inspiratory branch and a veterinary expiratory branch,
wherein the driving gas circuit comprises a gas driving device configured to force a driving gas into the veterinary inspiratory branch;
the fresh gas circuit comprises an anesthetic evaporation tank, a fresh gas carries an anesthetic gas in the anesthetic evaporation tank to the veterinary inspiratory branch via the fresh gas circuit, to be mixed with the driving gas to form a mixed gas which is then delivered to a patient side; and
the anesthetic gas recovery branch is connected to the veterinary expiratory branch, a waste anesthetic gas exhaled by the patient side enters a waste anesthetic gas recovery branch via the veterinary expiratory branch, and the waste anesthetic gas recovery branch comprises a waste anesthetic gas absorption tank and a waste gas absorption tank supporting device;
wherein the waste gas absorption tank supporting device comprises:
a tray provided with a tray recess; and
a protrusion structure provided on the tray, the protrusion structure being capable of forming a circumferential protective structure located on an outer side of the tray recess and configured to protect the waste gas absorption tank, and an inner side surface and an outer side surface of the protrusion structure being both protective surfaces capable of protecting the waste gas absorption tank.

21. A waste gas absorption tank supporting device, comprising:
a tray provided with a tray recess; and
a protrusion structure provided on the tray, the protrusion structure being capable of forming a circumferential protective structure located on an outer side of the tray recess and configured to protect a waste gas absorption tank, and an inner side surface and an outer side surface of the protrusion structure being both protective surfaces capable of protecting the waste gas absorption tank.

22. The waste gas absorption tank supporting device according to claim 21, wherein the protrusion structure is an annular flange.

23. The waste gas absorption tank supporting device according to claim 22, wherein an outer side surface of the annular flange is aligned with an outer circumferential surface of the tray.

24. The waste gas absorption tank supporting device according to claim 22, wherein there is a predetermined dimension between an outer side surface of the annular flange and an outer circumferential surface of the tray.

25. The waste gas absorption tank supporting device according to claim 21, wherein a plurality of protrusion structures are provided and arranged in a circumferential direction of the tray.

26. The waste gas absorption tank supporting device according to claim 21, wherein the protrusion structure is made of a flexible material.

27. The waste gas absorption tank supporting device according to any one of claims 21-26, wherein the protrusion structure is fixed relative to the tray.

28. The waste gas absorption tank supporting device according to claim 27, wherein the protrusion structure is fixedly connected to the tray by assembly.

29. The waste gas absorption tank supporting device according to claim 28, wherein the protrusion structure is provided with a mounting hole, and the tray is provided with a threaded hole; and
a fixing screw is further comprised, the fixing screw passing through the mounting hole to be in thread engagement with the threaded hole.

30. The waste gas absorption tank supporting device according to claim 28, wherein a connecting block is provided on the tray; and
an inner wall of the connecting block close to the center of the tray is an inner side wall of the tray recess, and the protrusion structure is a part of the connecting block away from the center of the tray.

31. The waste gas absorption tank supporting device according to claim 30, wherein the connecting block is provided with a stepped hole;
the stepped hole comprises a primary inner hole and a secondary inner hole, and the primary inner hole has a smaller dimension than the secondary inner hole;
a hole wall of the primary inner hole is the inner side wall of the tray recess; and a hole wall of the secondary inner hole, a top wall of the connecting block, and an outer wall of the connecting block form the protrusion structure.

32. The waste gas absorption tank supporting device according to claim 31, wherein a counter bore is formed in a connection plane between the primary inner hole and the secondary inner hole, and a threaded hole is formed in the tray; and
a fixing screw is further comprised, the fixing screw passing through the counter hole to be in thread engagement with the threaded hole.

33. The waste gas absorption tank supporting device according to claim 30, wherein a plurality of connecting blocks are provided and arranged in a circumferential direction of the tray;
each connecting block comprises a primary face and a secondary face that are arranged in a direction away from the center of the tray, the primary face and the secondary face being located on a side of the connecting block facing away from the tray;
each connecting block further comprises a primary facade connected to an end of the primary face away from the secondary face, and a secondary facade connecting the primary face to the secondary face, wherein the primary facade and the secondary facade face the center of the tray;
the primary facade is the inner side wall of the tray recess; and
the secondary facade, the secondary face and an outer wall of the connecting block form the protrusion structure.

34. The waste gas absorption tank supporting device according to claim 33, wherein a counter bore is formed in the primary face, and a threaded hole is formed in the tray; and
a fixing screw is further comprised, the fixing screw passing through the counter hole to be in thread engagement with the threaded hole.

35. The waste gas absorption tank supporting device according to claim 27, wherein the protrusion structure and the tray are integrated with each other.

36. The waste gas absorption tank supporting device according to any one of claims 21-26, wherein the protrusion structure is movably arranged on the tray.

37. The waste gas absorption tank supporting device according to claim 36, wherein a plurality of sliding slots are provided in the tray, and extension directions of the sliding slots are oriented in a radial direction of the tray; and
a plurality of protrusion structures are provided and arranged in a circumferential direction of the tray, and the protrusion structures are in one-to-one sliding fit with the sliding slots.

38. The waste gas absorption tank supporting device according to claim 37, wherein a connecting block is provided on the tray;
the connecting block comprises a primary face and a secondary face that are arranged in a direction away from the center of the tray, the primary face and the secondary face being located on a side of the connecting block facing away from the tray;
the connecting block further comprises a primary facade connected to an end of the primary face away from the secondary face, and a secondary facade connecting the primary face to the secondary face, wherein the primary facade and the secondary facade face the center of the tray;
the primary facade is the inner side wall of the tray recess; and
the secondary facade, the secondary face and an outer wall of the connecting block form the protrusion structure.

39. The waste gas absorption tank supporting device according to claim 38, wherein a counter bore is formed in the primary face; and
a sliding bolt mounted in the counter bore is further comprised, and a stud of the sliding bolt is slidably disposed in the corresponding sliding slot.
